(19) Europäisches Patentamt / European Patent Office / Office européen des brevets

(11) **EP 2 558 013 B1**

(12) **EUROPÄISCHE PATENTSCHRIFT**

(45) Veröffentlichungstag und Bekanntmachung des Hinweises auf die Patenterteilung:
**10.12.2014   Patentblatt 2014/50**

(21) Anmeldenummer: **11717517.4**

(22) Anmeldetag: **08.04.2011**

(51) Int Cl.:
*A61B 18/02* (2006.01)      *A61M 19/00* (2006.01)
*F25D 3/10* (2006.01)

(86) Internationale Anmeldenummer:
**PCT/EP2011/055486**

(87) Internationale Veröffentlichungsnummer:
**WO 2011/141243 (17.11.2011 Gazette 2011/46)**

(54) **HANDGEFÜHRTE KÜHLVORRICHTUNG ZUR KRYOTHERAPIE**

HAND-OPERATED COOLING DEVICE FOR CRYOTHERAPY

DISPOSITIF DE REFROIDISSEMENT À COMMANDE MANUELLE POUR CRYOTHÉRAPIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.04.2010   DE 102010016458**

(43) Veröffentlichungstag der Anmeldung:
**20.02.2013   Patentblatt 2013/08**

(73) Patentinhaber:
  • **Müller, Stefan**
    **78549 Spaichingen (DE)**
  • **Ortlib, Steffen**
    **78549 Spaichingen (DE)**
  • **Dreher, Ingo**
    **78582 Balgheim (DE)**

(72) Erfinder:
  • **Müller, Stefan**
    **78549 Spaichingen (DE)**
  • **Ortlib, Steffen**
    **78549 Spaichingen (DE)**
  • **Dreher, Ingo**
    **78582 Balgheim (DE)**

(74) Vertreter: **Spachmann, Holger et al**
    **Stumpf Patentanwälte PartGmbB**
    **Alte Weinsteige 71**
    **70597 Stuttgart (DE)**

(56) Entgegenhaltungen:
DE-C2- 19 548 652      DE-U1- 20 305 694
US-A- 2 536 001        US-A- 2 709 431
US-A- 2 746 264        US-A1- 2006 178 716
US-A1- 2008 142 036

EP 2 558 013 B1

**Beschreibung**

Hintergrund der Erfindung

Die vorliegende Erfindung geht aus von einer handgeführten Kühlvorrichtung zur Kryotherapie für die Behandlung von Schmerzen, Entzündungen, Muskelzerrungen oder dergleichen, umfassend einen Grundkörper und einen in den Grundkörper zumindest teilweise einsetzbaren Aufnahmebehälter zur auswechselbaren Aufnahme und Einsetzung eines Gasdruckbehälters.

STAND DER TECHNIK

Als Kryotheraphie bezeichnet man den gezielten Einsatz von Kälte, um einen therapeutischen Effekt zu bewirken. Insbesondere bei inneren Muskel-, Faser- oder Sehnenverletzungen, wie sie beispielsweise beim Sport auftreten können, kann eine Kühlung der betroffenen Stelle von außen mittels einer Kühlvorrichtung eine rasche Linderung bewirken, sowie einen Heilungsprozess beschleunigen. Dabei ist es wichtig, möglichst rasch nach dem Auftreten der Verletzung die betroffene Stelle äußerlich zu kühlen. Dabei ist es besonders vorteilhaft, die Epidermis, d.h. die Oberhaut, des zu behandelnden Bereichs auf eine Temperatur von etwa 2 bis 5°C abzukühlen, bei der die Kälte maximal auf die Hautrezeptoren einwirkt und hierbei schmerzstillend sowie durchblutungshemmend wirkt, um eine rasche Abheilung einer innerlich verletzten Stelle zu bewirken.

Die meisten Kryotheraphiegeräte verwenden als Kältequelle entweder gekühlte Luft oder flüssigen Stickstoff, der unmittelbar auf die betroffene Stelle aufgesprüht wird, was einen sehr unangenehmen Effekt bewirkt, da eine plötzliche, großflächige Kälteeinwirkung erzielt wird, die nur schlecht dosierbar ist und die durch Kondensation feuchte Hautpartien erzeugen kann. Des Weiteren ist es bekannt, unter Niederdruck stehende Gaspatronen zu verwenden, die eine Entspannung in unmittelbarer Umgebung eines lokalen Traumas mit einer Kälte von bis zu -25°C bewirken, wobei ein solcher Temperaturunterschied lokale Kälteverbrennungen hervorrufen kann.

Aus der DE 601 09 801 T2 ist ein handgeführtes Kryotherapiesprühgerät bekannt, bei dem Gas, beispielsweise $CO_2$, aus einem Gasdruckbehälter direkt auf einer zu kühlenden Stelle der betroffenen Epidermis aufgesprüht wird. Eine solche Kältesprühvorrichtung hat den Vorteil, dass eine sofortige Kühlungswirkung eintreten kann, da das expandierende Gas direkt auf die zu kühlende Stelle geleitet wird. Wird das Aufsprühen aus dem Gasdruckbehälter beendet, ist die Kühlwirkung allerdings sofort aufgehoben. Dabei wird die zu kühlende Stelle aufgrund der Kondensationswirkung des Gases sehr stark befeuchtet, da das expandierende Gas in einen flüssigen Aggregatzustand übergeht. Bei zu hohem Gasaustritt besteht die Gefahr von Kälteverbrennungen. Um eine lang andauernde Kühlwirkung zu gewährleisten, muss ein sehr großer Gasdruckbehälter mitgeführt werden, der die Abmessungen der Kühlvorrichtung vergrößert und somit eine flexible Anwendung und leichte Mitführbarkeit nachteilig behindert.

Die US 2009 02 480 01 A1 offenbart eine handgeführte Kühlvorrichtung, wobei eine Sondennadel durch die Oberfläche einer Haut gestoßen wird. Ein Kühlfluid wird mittels eines elektrischen Steuerungssystems zur Kühlung durch die Sondennadel geleitet. Das Kühlfluid kann durch eine $N_2O$-Kartusche bereitgestellt werden.

In der DE 699 18 810 T2 wird eine Kühlvorrichtung beschrieben, die eine $CO_2$-Patrone enthält, wobei das $CO_2$-Gas aus einer Öffnung der Kühlfläche austreten kann. Die Kühlvorrichtung wird mittels eines elektrischen Ventils betätigt und die Patrone ragt aus der Vorrichtung heraus..

Aus der DE 195 48 652 A1 ist eine Kühlvorrichtung mit einer $CO_2$-Kapsel bekannt, bei der Kälte durch ausströmendes Gas mittels eines Wärmetauschers appliziert werden kann. Die Vorrichtung umfasst elektronische Bauteile wie Digitalthermometer. Die Kapsel ist nicht im Gehäuseinneren der Vorrichtung untergebracht.

Schließlich wird in der DE 203 05 694 U1 eine Kühlvorrichtung mit einer einsetzbaren Gaskartusche offenbart, aus der durch mechanische Betätigung eines Ventils ein Kühlgas austreten kann. Das austretende Kühlgas kann auf einen zu kühlenden Hautbereich gerichtet werden.

Die US 2 536 001 A beschreibt eine Kühlvorrichtung mit einer ein Kühlmittel beinhaltenden Kartusche, die einen Aufnahmebehälter und einen Grundkörper mit Kühlmittelaustrittsbetätigungsmittel umfasst. Das Kühlmittel entspannt sich im Inneren der Kühlmittelvorrichtung und kühlt eine Kühlfläche, die an einem axialen Ende des Grundkörpers angeordnet ist. Das Kühlgas tritt an einer Austrittsfläche des Aufnahmebehälters aus, die axial entgegengesetzt zur Kühlfläche des Grundkörpers angeordnet ist. Demgemass weist die Kühlvorrichtung eine relativ große Baugröße auf, da das Kühlgas im Inneren entspannt, um die Kühlfläche zu kühlen, die weit von der Gasaustrittsstelle entfernt liegt.

Aus der DE 195 48 652 C geht eine Kühlvorrichtung für eine Kältetherapie hervor, bei der Gas zur Kühlung auf die Haut abgegeben werden kann, wobei die Gasaustrittszeit und der Abstand der Gasaustrittsdüse von der Haut elektronisch einstellbar ist.

Durch die DE 203 05 694 U ist ein weiteres gattungsgemäßes Kryotherapiegerät bekannt, dass ein Gasaustrittsbetätigungsmittel zur direkten Einwirkung von Kühlmittel auf die Haut aufweist, wobei zur Betätigung des Gasaustrittsbetätigungsmittels ein Sicherungsmittel zu entriegeln ist.

In der US 2008 142036 A geht eine Kühlvorrichtung hervor, in der eine Kühlfläche eines Grundkörpers aus Metall und ein Aufnahmebehälter aus thermisch isolierten Material wie Kunststoff mit einer thermischen Leitfähigkeit unter 1 Wm$^{-1}$K$^{-1}$ besteht. Aufgabe der vor-

liegenden Erfindung ist es daher, eine mobil einsetzbare handgeführte Kühlvorrichtung vorzuschlagen, die keine direkte Gasabgabe auf eine zu kühlende Stelle bewirkt, dabei eine falsche Handhabung oder die Gefahr einer Gefrierverbrennung verhindert, eine konstante, lang andauernde Kühlwirkung bereitstellt und dabei nur geringe Abmessungen aufweist und transportabel ausgestaltet ist. Des Weiteren ist es Aufgabe der Kühlvorrichtung, eine Kühlwirkung mittels Gasdruckbehälter zu erreichen, die in hoher Stückzahl zu geringen Kosten verfügbar sind, um die Lebensdauer und die Betriebskosten der Kühlvorrichtung gering zu halten.

OFFENBARUNG DER ERFINDUNG

Die Erfindung wird im Anspruch 1 definiert.
Die vorliegende Erfindung geht aus von einer handgeführten Kühlvorrichtung zur Kryotherapie für die Behandlung von Schmerzen, Entzündungen, Muskelzerrungen oder dergleichen, umfassend einen Grundkörper und einen in den Grundkörper zumindest teilweise einsetzbaren Aufnahmebehälter zur auswechselbaren Aufnahme und Einsetzung eines Gasdruckbehälters. Die vorliegende Erfindung schlägt vor, dass der Grundkörper ein Gasaustrittsbetätigungsmittel umfasst, das manuell betätigbar, ein Ausströmen eines Gases aus dem Gasdruckbehälter bei eingesetztem Aufnahmebehälter bewirken kann und der Aufnahmebehälter eine Kühlfläche umfasst, die durch das ausströmende Gas sowie durch den Gasdruckbehältermantel kühlbar ist, wobei das Gas innerhalb des Grundkörpers und des Aufnahmebehälters mittels Gasführungsmittel geführt und am Aufnahmebehälter benachbart zur Kühlfläche austreten kann. Mit anderen Worten schlägt die vorliegende Erfindung vor, dass eine zumindest zweiteilig aufgebaute Kühlvorrichtung aus einem Grundkörper besteht, der ein Gasaustrittsbetätigungsmittel umfasst, durch das das Austreten eines Gases aus einem Gasdruckbehälter bewirkt werden kann. Der Gasdruckbehälter wird in einen Aufnahmebehälter aufgenommen und dieser dann in den Grundkörper eingesetzt, so dass bei Betätigung des Gasaustrittsbetätigungsmittels das Gas des Gasdruckbehälters austritt und mittels Gasführungsmittel im Inneren des Grundkörpers und des Aufnahmebehälters so geführt wird, dass es benachbart zu einer Kühlfläche, die sich am Aufnahmebehälter befindet, insbesondere die Bodenfläche des Aufnahmebehälters bildet, ausströmen kann, um die Kühlfläche zu kühlen. Dabei ist wesentlich, dass das ausströmende Gas nicht in Richtung der Kühlfläche abgegeben wird, sondern seitlich oder nach oben weg von der Kühlfläche abgegeben wird, um die kühlende Fläche nicht unmittelbar zu begasen bzw. zu besprühen. Durch die Expansionswirkung des Gases aus dem Gasdruckbehälter entsteht zum einen durch die Expansionswirkung entlang des Gasführungsmittels Kälte, zum anderen wird der Gasdruckbehälter durch die Entspannung stark herabgekühlt, wobei der Gasdruckbehälter zumeist aus Metall, insbesondere Aluminium oder ähnlichem Material, besteht. Entweicht das Gas aus dem Gasdruckbehälter, wird entsprechend der Formel

$$\frac{P \cdot V}{T} = \mathrm{konstant}$$ der Druck P innerhalb des Gasdruckbehälters bei gleichbleibendem Volumen V erheblich vermindert. Demzufolge muss, da das Volumen konstant bleibt, die Temperatur T ebenfalls verringert werden, wodurch sich der Gasdruckbehälter stark abkühlt. Durch das Abkühlen des Gasdruckbehälters und die Gasentspannung des Gases im Inneren der Kühlvorrichtung wird Kälte erzeugt, die von dem Aufnahmebehälter, der hierzu bevorzugt aus einem gut leitendem Wärmematerial wie beispielsweise einem Metall besteht, aufgenommen und somit die auf der Außenfläche angeordnete Kühlfläche gekühlt. Je nach Wärmespeicherkapazität des Aufnahmebehälters und des Gasdruckbehälters kann eine beliebige Kältemenge gespeichert und an der Kühlfläche abgegeben werden. Je nach Regelung der Austrittsgeschwindigkeit des Gases kann gezielt die Temperaturänderung und damit die sich an der Kühlfläche einstellende Temperatur vorbestimmt werden, so dass insbesondere eine vorteilhafte Kühltemperatur von 2 bis 5°C an der Kühlfläche erreicht werden kann. Bei hoher Wärmekapazität der Gesamtanordnung kann diese Kühlwirkung über lange Zeit aufrecht erhalten werden, beispielsweise in einem Zeitraum von 3 bis 10 Minuten. Das expandierende Gas wird nicht direkt auf die zu kühlende Stelle, sondern indirekt abgegeben und dient lediglich zur Druckentspannung des Gasdruckbehälters und zum Herunterkühlen der gesamten Kühlvorrichtung bzw. der Kühlfläche des Aufnahmebehälters. Wird das Gasaustrittsbetätigungsmittel am Grundkörper betätigt kann in einem Zeitraum von 2 bis 30 Sekunden das Gas austreten, das in der Nähe der Kühlfläche indirekt abgegeben wird und die Kühlfläche bzw. der gesamte Aufnahmebehälter kann auf eine Temperatur von ca. 2 bis 10°C abgekühlt werden, wobei diese Temperatur über einen Zeitraum von 3 bis 5 Minuten gehalten werden kann. Durch den lediglich zweiteiligen Aufbau der Kühlvorrichtung und einem einfachen Einsetzen eines Gasdruckbehälters in den Aufnahmebehälter, der in den Grundkörper eingesetzt bzw. eingeschraubt werden kann, kann die Vorrichtung sehr einfach von einem Laien bedient werden. Die Abmessungen der Kühlvorrichtung können sich nur unwesentlich von den Abmessungen des benutzen Gasdruckbehälters unterscheiden, so dass bei Verwendung eines kleinen Gasdruckbehälters, beispielsweise eine 8g-Sahnekapsel, einen sehr kleinen "Kühlstick" ergibt, der transportabel überall mitgenommen und flexibel eingesetzt werden kann. Somit ist die vorgeschlagene Kühlvorrichtung sehr klein und die Handhabung einfach, wobei die Gefahr von Kälteverbrennungen unterbunden ist. Das Gerät erreicht somit eine hohe Mobilität, kann platzsparend und nachfüllbar eingesetzt werden und erzeugt eine konstante Kälteleistung über einen langen Zeitraum. Die Kühlvorrichtung ist für eine Vielzahl von Therapieanwendungen ausgelegt und weist eine hohe Lebensdauer auf.

Gemäß einer vorteilhaften Ausgestaltung ist der Aufnahmebehälter im Wesentlichen becherförmig und formkomplementär zu dem Bodenbereich des Gasdruckbehälters ausgestaltet und aus einem Material hoher Wärmeleitfähigkeit, bevorzugt Metall ausgebildet, wobei die Bodenaußenfläche des Aufnahmebehälters die Kühlfläche ausbildet. Des Weiteren kann der Aufnahmebehälter zumindest einen, insbesondere mehrere Gasaustrittskanäle entlang eines Mantelumfangbereichs am bodenseitigen Ende umfassen und der Aufnahmebehälter den Gasdruckbehälter zumindest im Bodenbereich im Wesentlichen formschlüssig zur Erzielung einer guten Wärmekopplung aufnehmen, wobei zwischen Innenwand des Aufnahmebehälters und äußerem Gasdruckbehälter ausströmendes Gas vom offenen, im Grundkörper eingesetzten Ende des Aufnahmebehälters bis zu dem Gasaustrittskanal oder -kanälen bevorzugt entlang von einem oder mehreren Gasführungsmitteln, insbesondere Gasführungskanälen, strömen kann. Somit betrifft diese Weiterbildung eine vorteilhafte Ausgestaltung des Aufnahmebehälters, der aus einem Material hoher Wärmeleitfähigkeit, bevorzugt Metall besteht. Der Aufnahmebehälter kann vorteilhaft becherförmig und formkomplementär zur Unterseite (Bodenbereich) eines Gasdruckbehälters, insbesondere einer marktgängigen Sahnekapsel oder einer Patrone für einen $CO_2$-Wasserspender, ausgestaltet sein, wobei die Bodenaußenfläche des Aufnahmebehälters gleichzeitig als Kühlfläche ausgebildet ist. Somit kann bei eingesetztem Aufnahmebehälter in den Grundkörper diese Bodenfläche, die ein axiales Ende der im Wesentlichen zylinderförmigen Kühlvorrichtung bildet, auf eine zu behandelnde Stelle aufgesetzt werden. Die Bodenfläche kann dabei plan, spitz oder auch abgerundet ausgebildet sein, um entsprechend großflächige oder punktuelle Kältewirkungen zu erzeugen. Insbesondere eine abgerundete Kühlfläche kann bei nachgiebigem Gewebe je nach Anpressdruck sowohl zu punktuellen als auch zur großflächigen Kühlung genutzt werden. Die Gasführungsmittel können beispielsweise Gasführungskanäle sein, die im Inneren der Außenwandung des Aufnahmebehälters verlaufen, jedoch könnte das Gasführungsmittel auch ein Spalt zwischen Gasdruckbehältermantel und Innenfläche des Aufnahmebehälters umfassen, wobei das Gas entlang der Gasdruckmantelfläche des Gasdruckbehälters strömen und in der Nähe der Kühlfläche am bodenseitigen Ende des Aufnahmebehälters durch Gasaustrittskanäle austreten kann. Dabei ist zu beachten, dass die Gasaustrittskanäle das Gas so aus der Kühlvorrichtung ableiten, dass keine unmittelbare Sprühwirkung auf die zu kühlende Epidermis, auf die der Kühlstick angewendet wird, ausgeübt wird. Das Gas tritt im Inneren des Grundkörpers, der ein Gasaustrittsbetätigungsmittel beispielsweise einen penetrierenden Stift, der eine Verschlusskappe des Gasdruckbehälters durchstößt aus, wobei das Gas im Inneren des Grundkörpers in das oben offene Ende des Aufnahmebehälters geleitet und dann innerhalb des Aufnahmebehälters weiter bis zum bodenseitigen Ende des Aufnahmebehälters weitergeleitet wird und dort austreten kann. Durch die becherförmige Ausgestaltung des Aufnahmebehälters kann ein sehr einfacher und ohne besondere Anleitung zu beachtender Einsatz oder Auswechslung von Gasdruckbehältern für die Kühlvorrichtung vorgeschlagen werden.

Gemäß einer weiteren vorteilhaften Ausgestaltung kann der Aufnahmebehälter mit aufgenommenem Gasdruckbehälter in den Grundkörper eingeschraubt, eingerastet oder bajonettverschlussartig eingesetzt werden. Nach Aufnahme eines Gasdruckbehälters in den bevorzugt becherförmigen Aufnahmebehälter muss dieser zur Aktivierung der Kühlwirkung in den Grundkörper eingesetzt werden, wobei der Grundkörper mit Gasaustrittsbetätigungsmittel bewirkt, dass das Gas ausströmt und den Gasdruckbehältermantel abkühlt und diese Abkühlwirkung über den Außenmantel des Aufnahmebehälters, insbesondere die Kühlfläche nach außen auf eine zu behandelnde Stelle abgegeben werden kann. Die Verbindung von Aufnahmebehälter und Grundkörper kann mittels einer Schraubverbindung oder einer Rastverbindung erfolgen, in der beispielsweise eine wiederöffenbare Rastverbindung zwischen Aufnahmebehälter und Grundkörper hergestellt werden kann. Schließlich ist auch ein Bajonett-Verschluss denkbar, bei dem in einer schräg verlaufenden Nut des Grundkörpers oder des Aufnahmebehälters Führungsnasen des Gegenstücks eingebracht, verdreht und in eine fixierende Position gebracht werden. Wesentlich ist, dass die Verbindungsmechanik zwischen Aufnahmebehälter und Grundkörper ausgelegt ist, hohe Kräfte aufzunehmen, da bei der Gasentspannung hohe Druckentweichungskräfte auftreten und bei Drosselung des Gasaustritts diese Kräfte durch kraftschlüssige Verbindung zwischen Aufnahmebehälter und Grundkörper aufgefangen werden müssen. Eine nicht gesicherte Verbindung zwischen Aufnahmebehälter und Grundkörper kann zu einer explosionsartigen Abstoßung der beiden Teile der Kühlvorrichtung führen, die unbedingt zu vermeiden ist.

Gemäß einer weiteren vorteilhaften Ausgestaltung kann das Gasaustrittsbetätigungsmittel eine Dreh-, Wipp-, Druck-, oder Zugaustrittsmechanik zum Bewirken eines Gasaustritts aus dem Gasdruckbehälter umfassen, wobei nach eingesetztem Gasdruckbehälter durch eine Dreh-, Wipp-, Druck-, oder Zugbewegung eines Betätigungselements die Austrittsmechanik ein Verschlussventil des Gasdruckbehälters oder des Grundkörpers betätigen kann, oder eine Verschlussmembran des Gasdruckbehälters durchstoßen kann. So kann in diesen beiden alternativen Ausführungsformen des Gasaustrittsbetätigungsmittels bei Einsetzen des Aufnahmebehälters in den Grundkörper bereits eine Öffnung des Gasdruckbehälters erfolgen, wodurch Gas in das Innere der Kühlvorrichtung geleitet wird. Hier kann für eine gasdichte Ausführung der Gasführungsmittel ein Verschlussventil angeordnet sein, wobei durch Betätigung des Verschlussventils das Gas mittels Gasführungsmitteln entlang des Gasdruckbehälters geleitet und durch Gasführungskanäle abgegeben werden kann, um eine Kühlwirkung zu erzielen. Alternativ hierzu kann durch Betätigung des Gasaustrittsbetätigungsmittels eine zuvor geschlossene Membran oder ein am Gasdruckbehälter angeordnetes Ventil betätigt werden, um den Gasaustritt zu bewirken. Das Gasaustrittsbetätigungsmittel kann somit entweder ein Verschlussventil zur Regelung der Menge des ausströmenden Gases und/oder ein Öffnungsmittel zur Öffnung einer Verschlussmembran des Gasdruckbehälters umfassen. Des Weiteren kann das Gasaustrittsbetätigungsmittel eine Austrittsmechanik, die beispielsweise als Dreh-, Wipp-, Druck-, oder Zugmechanik ausgestaltet ist, um das entsprechende Verschlussventil oder das entsprechende Öffnungselement zum Durchstoßen der Verschlussmembran zu betätigen, umfassen. Beispielsweise kann das Gasaustrittsbetätigungsmittel eine Schraubverbindung umfassen, bei der durch Drehen ein Öffnungsstiftelement eine Verschlussmembran des Gasdruckbehälters durchstößt oder ein Gasaustrittsventil betätigt. Statt einer Drehwirkung kann durch eine Druckwirkung auf eine Druckkappe ein gleichsames Öffnen des Gasaustrittsventils ermöglicht werden. Alternativ kann durch eine Wippmechanik oder eine Zugmechanik ebenfalls ein Durchstoßen der Verschlussmembran oder ein Betätigen eines Verschlussventils oder beides ermöglicht werden.

Gemäß einer weiteren vorteilhaften Weiterentwicklung kann das Gasaustrittsbetätigungsmittel einen Öffnungsstift zum penetrierenden Durchstoßen der Verschlussmembran des Gasdruckbehälters umfassen. Dieser Öffnungsstift kann insbesondere in einem Öffnungsstiftelement untergebracht sein und durch die vorgenannte Öffnungsbewegung ein penetrierendes Durchstoßen einer Verschlussmembran eines Gasdruckbehälters, insbesondere einer konventionellen Sahnekapsel oder Luftdruckpatrone, bewirken.

Grundsätzlich kann der Gasaustritt nach Betätigung des Gasaustrittsbetätigungsmittels instantan und ungehindert erfolgen, so dass eine hohe Abkühlwirkung durch ein vollständiges Entströmen des Gases erreicht wird. Bevorzugt kann in einer vorteilhaften Weiterbildung der Erfindung zumindest eine Gasdurchtrittsblende im Grundkörper umfasst sein, die ausgelegt ist, die Gasaustrittsgeschwindigkeit zu drosseln, wobei bevorzugt die Gasdurchtrittsblende einen einstellbaren Blendendurchmesser d aufweist, so dass die Gasaustrittsgeschwindigkeit und somit die gewünschte Kühltemperatur bevorzugt im Bereich zwischen 2 bis 12°C, insbesondere 5 bis 7°C, einstellbar ist. Eine solche Gasdurchtrittsblende wirkt als Gasströmungsdrossel und drosselt die Geschwindigkeit, mit der das Gas aus der Gasdruckpatrone ausströmen kann. Durch gezielte Beeinflussung der Strömgeschwindigkeit kann die Zeitdauer bis zur vollständigen Entleerung des Gasdruckbehälters eingestellt werden, wodurch die zu erreichende Endtemperatur und die Dauer des Kühlvorgangs unmittelbar beeinflussbar sind. Eine solche Blende kann einen fixen Durchmesser d aufweisen, der für die genannten Temperaturbereiche ausgelegt werden kann. Allerdings ist es auch vorstellbar, den Blendendurchmesser d variabel einstellbar auszulegen, so dass je nach Anwendungsfall durch Regulierung der Gasausströmmenge eine hohe (kurze) oder niedrige (lang andauernde) Kühlwirkung erzielt werden kann. Des Weiteren ist es auch vorstellbar, bei Anwendung eines Verschlussventils oder ein als Ventil fungierende verschließbare Blende nicht das gesamte Gas ausströmen zu lassen, so dass insbesondere bei niedrigen Strömungsgeschwindigkeiten ein mehrmaliger Einsatz der Kühlwirkung mit einem einzigen Gasdruckbehälter erreicht werden kann.

Entsprechend einer vorteilhaften Weiterbildung der Erfindung kann die Austrittsmechanik des Gasaustrittsbetätigungsmittels ein Sicherungselement umfassen, das eine ungewollte Betätigung des Betätigungselements verhindern kann. Ein solches Sicherungselement verhindert insbesondere beim transportablen Einsatz, beispielsweise in einer Notfalltasche oder unter schwierigen Bedingungen, wie beispielsweise beim Einsatz im Sport, auf einer Reise etc. ein ungewolltes Auslösen des Gasaustrittsbetätigungsmittels und damit ein ungewolltes Verbrauchen der einmalig abrufbaren Kühlwirkung der Kühlvorrichtung. Ein solches Sicherungsmittel kann beispielsweise ein Sicherungsstift oder ein Sicherungsdruckknopf sein, der eine arretierte Position der Austrittsmechanik sicherstellt, so dass diese nicht ungewollt ohne Betätigen des Sicherheitsknopfes bzw. Herausziehen des Sicherheitsstiftes aktivierbar ist. Somit wird eine robuste Handhabung ermöglicht und eine Fehlbedienung der Kühlvorrichtung verhindert.

Entsprechend einer vorteilhaften Weiterbildung der Erfindung kann der Aufnahmebehälter geeignet sein, einen $N_2O$-(Lachgas) oder $CO_2$- (Kohlendioxid)-Gasdruckbehälter mit einem Füllgewicht von 2 bis 350g, bevorzugt von 6 bis 20g, insbesondere von 8g aufzunehmen, der eine Längenabmessung von 4 bis 20cm, insbesondere 6 bis 7cm und einen Durchmesser von 1 bis 7cm, insbesondere 1 bis 2,5cm aufweisen kann. Somit schlägt diese Weiterbildung vor, einen handelsüblichen $N_2O$- oder $CO_2$-Gasdruckbehälter, wie er beispielsweise beim Einsatz von Wassersprudlern, bei der Sahneaufschäumung, im Gasdruck-Schießsport oder bei der Kühlung von Getränkeanlagen verwendbar ist, in universell einsetzbaren und in großen Mengen kostengünstig verfügbaren Behältern einzusetzen, wobei das Füllgewicht des Gasdruckbehälters unmittelbar die erreichbare Kühltemperatur und Kühldauer beeinflusst. Je größer der Gasdruckbehälter, umso tiefer kann die erzielbare Temperatur und/oder umso länger kann die erzielbare Kühlwirkung andauern. Insbesondere kleine Gasdruckbehälter von 8g, die in der Regel eine Länge von 5 bis 7cm und einen Durchmesser von 1 bis 2cm aufweisen, sind hervorragend für den Einsatz in der handgeführten Kühlvorrichtung geeignet, da damit geringe Abmessungen der Kühlvorrichtung ermöglicht werden, so dass diese transportabel, beispielsweise in der Hosentasche oder in einem Notfallset, kompakt mitgeführt und mobil eingesetzt werden können.

Besonders bevorzugt kann der Gasdruckbehälter eine handelsübliche $CO_2$-8g-Sahne-spenderpatrone, insbesondere eine in großen Mengen zu günstigen Preisen auf dem Markt verfügbare ISI-Sahnekapsel sein. Eine Sahnekapsel ist praktisch überall in Supermärkten frei verfügbar und kann kostengünstig in die Kühlvorrichtung eingesetzt und mobil mitgeführt werden.

Betreffend die Materialwahl der Kühlvorrichtung kann der Grundkörper aus beliebigen Materialien ausgebaut sein, wobei insbesondere die Kühlfläche des Aufnahmebehälters aus einem Material hoher Wärmeleitfähigkeit bestehen sollte. Besonders bevorzugt ist zumindest die Kühlfläche des Ausnahmebehälters aus einem Werkstoff hoher spezifischer Wärmeleitfähigkeit, bevorzugt Metall, insbesondere einer Aluminium-Legierung, wie AlMgSi, aufgebaut und der Grundkörper sowie die übrigen Teile aus einem Material niedriger spezifischer Wärmeleitfähigkeit, d.h. hoher Isolationswirkung, insbesondere wärmeisolierenden Kunststoff, bevorzugt POM (Polyoxymethylen) aufgebaut, wobei bevorzugt ein Handführungsbereich des Grundkörpers zusätzlich durch eine Isolationsschicht wärmeisolierend ausgestaltet ist. Somit kann insbesondere der Grundkörper aus einem wärmeisolierenden Material, insbesondere einem günstigen Kunststoff, aufgebaut sein, wobei der Handführungsbereich besonders isoliert ist, um bei Anwendung der Kühlvorrichtung den Benutzer vor der Kältewirkung zu schützen. Die Kältewirkung soll ausschließlich an der Kühlfläche des Ausnahmebehälters nach außen treten, somit ist vorteilhaft diese Kühlfläche und das umgebende Material mit einer hohen Wärmeleitfähigkeit, insbesondere einer hohen elektrischen Leitfähigkeit, ausgestattet und weist ein großes Wärmespeichervolumen auf, so dass die Kühltemperatur über längere Zeit gespeichert und abgegeben werden kann. Somit schlägt diese Ausführung vor, dass weite Bereiche der Kühlvorrichtung wärmeisolierend ausgestaltet sind, so dass die erzeugbare Kälte, die sich insbesondere im Gasdruckbehältermantel ausbildet, nur über die Kühlfläche nach außen abgegeben werden kann, so dass eine effiziente und lang andauernde Kühlwirkung erreicht werden kann.

Bei Austritt des Gases aus dem Gasdruckbehälter entstehen hohe Gasströmgeschwindigkeiten und große Gasdruckkräfte. Um eine hohe Langlebigkeit der Kühlvorrichtung zu erreichen ist es vorteilhaft, die im Kühlstrom des Gasaustritts wirkenden Teile besonders widerstandsfähig auszubilden. Daher schlägt eine vorteilhafte Weiterbildung vor, dass der Öffnungsstift, insbesondere ein Öffnungsstiftelement, aus einem widerstandsfähigem Material, insbesondere Stahl, wie X20Cr13, besteht. Durch Ausbildung des Öffnungsstifts, der zum Durchstoßen einer Verschlussmembran eines Gasdruckbehälters oder zum Öffnen eines im Gasdruckbehälter integrierten Ventils dient, wird somit ein Öffnungsstiftelement vorgeschlagen, das geeignet ist, eine dicke Verschlussmembran zu durchstoßen oder ein schwergängiges Gasventil eines Gasdruckbehälters zu betätigen und das hohe Austrittskräfte und die erzielte Kühlwirkung im Inneren des Kühlstifts aufnehmen kann und für eine lange Lebensdauer und ein hohes Durchsatzvolumen an Gasaustritten besonders robust ausgestaltet ist.

Nach einem weiteren vorteilhaften Ausführungsbeispiel, das insbesondere für die konstruktive Auslegung der Kühlvorrichtung wesentlich ist und für eine hohe Lebensdauer, niedrige Produktionskosten, sowie gute Transportier- und Handhabbarkeit der Kühlvorrichtung ausgelegt ist, kann der Grundkörper mehrteilig ausgeführt sein und insbesondere eine Deckkappe, ein Griffstück, ein Aufnahmeelement, ein Verschlusselement, ein Glockenelement und ein Öffnungsstiftelement umfassen, wobei zumindest die Deckkappe, das Verschlusselement, das Glockenelement und das Öffnungsstift-element formschlüssig zur Ausbildung eines Gasaustrittsbetätigungsmittels miteinander verbunden sind. Des Weiteren kann ein Handführungselement zumindest ein Griffstück und ein Aufnahmeelement, die formschlüssig miteinander verbunden sind, umfassen, so dass das Handführungselement zur Aufnahme des Aufnahmebehälters und zur Handführung der Kühlvorrichtung durch einen Benutzer dient. Somit schlägt diese Weiterbildung vor, dass der Grundkörper grundsätzlich zweiteilig aufgebaut ist, wobei der eine Teil als Gasaustrittsbetätigungsmittel aufgebaut ist und mehrteilig mehrere funktional ineinander greifende und miteinander formschlüssig verbundene Teile umfasst. Des Weiteren umfasst der zweite Teil des Grundkörpers das Handführungselement, eine Aufnahmemechanik zur Aufnahme des Aufnahmebehälters und zur Weiterleitung des Gases in den Aufnahmebehälter nach Austritt im Gasaustrittsbetätigungsmittel. Dabei ist es besonders vorteilhaft, dass das Gasaustrittsbetätigungsmittel gegenüber dem Handführungselement verschieblich, insbesondere verdrehbar zur Ausübung einer Betätigungsbewegung für eine penetrierende Durchstoßung einer Verschlussmembran eines aufgenommenen Gasdruckbehälters bzw. zur Öffnung eines im Gasdruckbehälter befindlichen Ventils gelagert ist. Somit schlägt diese Weiterbildung vor, dass der Grundkörper im Wesentlichen zweiteilig aufgebaut ist und ein Gasaustrittsbetätigungsmittel als axiales Endstück, sowie ein Handführungselement zur Handführung und zur Aufnahme des Aufnahmebehälters umfasst. Diese beiden Teile umfassen wiederum mehrere funktional und strukturell ineinandergreifende Einzelkomponenten, die einem hohen Gasdruck standhalten und dabei wärmeisolierend ausgestaltet sind. Vorteilhaft kann zur Bewirkung eines Gasaustritts aus dem Gasdruckbehälter das Gasaustrittsbetätigungsmittel gegenüber dem Handführungselement verschieblich, insbesondere verdrehbar ausgeführt sein, um das Öffnungsstiftelement des Gasaustrittsbetätigungsmittels eines im Handführungselement aufgenommenen Gasdruckbehälters zu durchstoßen.

ZEICHNUNGEN

Weitere Vorteile ergeben sich aus der vorliegenden Zeichnungsbeschreibung. In der Zeichnung sind Ausführungsbeispiele der Erfindung dargestellt. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:

Fig. 1    eine Seitendarstellung und einen Schnitt durch ein erstes Ausführungsbeispiel einer handgeführten Kühlvorrichtung;

Fig. 2    einen Schnitt durch einen Gasdruckbehälter, der in eine erfindungsgemäße Kühlvorrichtung eingesetzt werden kann;

Fig. 3    verschiedene Darstellungen einer Deckkappe des ersten Ausführungsbeispiels;

Fig. 4    verschiedenen Darstellungen eines Verschlusselements des ersten Ausführungsbeispiels;

Fig. 5    verschiedene Darstellungen eines Glockenelements des ersten Ausführungsbeispiels;

Fig. 6    verschiedene Darstellungen eines Öffnungsstiftelements des ersten Ausführungsbeispiels;

Fig. 7    verschiedene Darstellungen eines Griffstücks des ersten Ausführungsbeispiels;

Fig. 8    verschiedene Darstellungen eines Aufnahmeelements des ersten Ausführungsbeispiels;

Fig. 9    verschiedene Ausführungen eines Aufnahmebehälters des ersten Ausführungsbeispiels;

Fig. 10   schematisch einen Schnitt durch ein zweites Ausführungsbeispiel einer Kühlvorrichtung;

Fig. 11   weitere schematische Darstellungen von Ausführungsbeispielen einer handgeführten Kühlvorrichtung

AUSFÜHRUNGSFORMEN DER ERFINDUNG

In den Figuren sind gleiche oder gleichartige Komponenten mit gleichen Bezugszeichen beziffert.

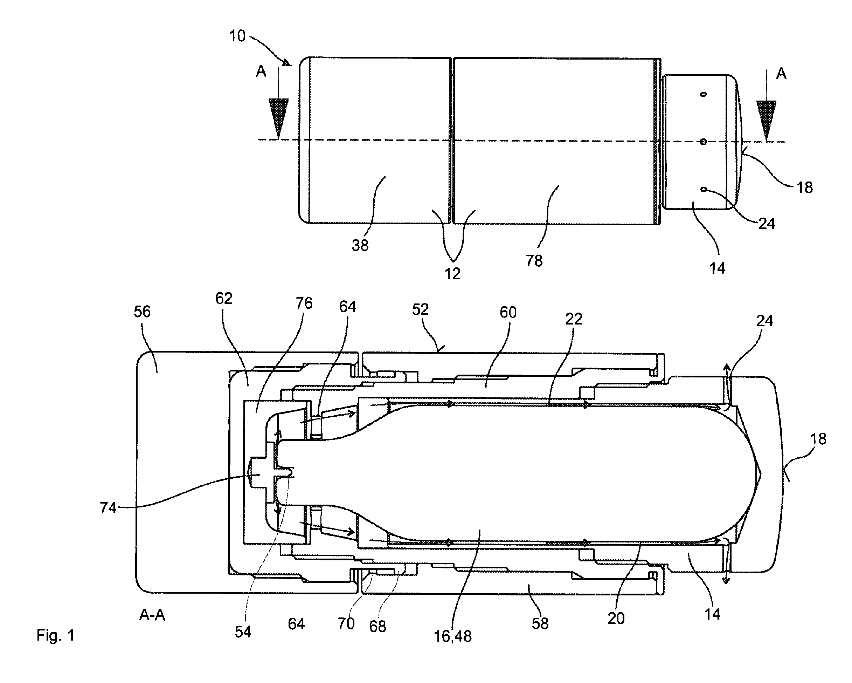

Fig. 1 stellt eine Seitenansicht sowie einen Schnitt A-A durch ein erstes Ausführungsbeispiel 10 einer handgeführten Kühlvorrichtung zur Kryotheraphie dar. Im Wesentlichen umfasst die Kühlvorrichtung 10 einen Grundkörper 12, der sich aus einem Betätigungselement 38 und einem Handführungselement 78 zusammensetzt, sowie einen Aufnahmebehälter 14, der zur Aufnahme eines Gasdruckbehälters 16, in diesem Fall einer Sahnekapsel 48, dient und der in den Grundkörper 12 eingesetzt, insbesondere eingeschraubt werden kann. Das Betätigungselement 38 des Grundkörpers 12 umfasst eine Deckkappe 56, ein Verschlusselement 62 sowie ein Glocken-element 76, das ein Öffnungsstiftelement 74 umfasst. Das Betätigungselement 38 ist an dem Handführungselement 78 mittels eines Feingewindes sowie Rastelemente 68, 70, insbesondere Rastringe drehbar angeordnet, so dass es längs-verschieblich mittels einer Drehbewegung axial hin und her bewegt werden kann, wobei die Bewegungsrichtung durch die Rastelemente 68, 70 in beide Richtungen limitiert ist. Das Handführungselement 78 umfasst ein Griffstück 58, sowie ein in das Griffstück eingeschraubtes Aufnahmeelement 60, das zur Aufnahme des Aufnahmebehälters 14 dient. Der Aufnahmebehälter 14 lagert bodenseitig eine Sahnekapsel 48 und ist mittels eines Feingewindes in das Aufnahmeelement 60 des Handführungselements 78 eingeschraubt. Wird nun das Betätigungselement 38 gegenüber dem Handführungselement 78 mittels einer Drehbewegung derart axial eingeschraubt, dass es sich in Richtung Handführungselement 78 bewegt, durchsticht ein Öffnungsstift 54 des Öffnungsstiftelements 74 eine Verschlussmembran 44 der Sahnekapsel 48 bzw. öffnet deren kopfseitiges Ventil. Hierbei tritt, wie durch Pfeile angedeutet, Gas aus der Sahnekapsel 48 aus und umströmt den Innenbereich des Glockenelements 76, strömt durch die Gasdurchtrittsblenden 64 des Aufnahmeelements 60 und strömt im Zwischenraum zwischen Innenoberfläche des Aufnahmeelements 60 und Gasdruckbehältermantel 20 in den bodenseitigen Bereich des Aufnahmebehälters 14, wo das Gas durch Gasführungskanäle 24 nach außen austreten kann, ohne auf die zu kühlende Stelle zu gelangen. Auf der Unterseite des becherförmigen Aufnahmebehälters 14 ist eine Kühlfläche 18 angeordnet. Der kontrollierte Austritt des Gases aus der Sahnekapsel 48 bewirkt ein verhältnismäßig starkes Abkühlen des Gasdruckbehältermantels 20, wobei das strömende Gas ebenfalls Kälte entlang der inneren Oberfläche des Aufnahmebehälters 14 erzeugt. Da der Grundkörper 12 aus einem wärmeisolierenden Kunststoff besteht, kann diese Kälte insbesondere im Handführungsbereich 52 nicht nach außen abgegeben werden, so dass insbesondere die Bodenseite und somit die Kühlfläche 18 des aus Metall, insbesondere Aluminium, bestehenden Aufnahmebehälters 14 sehr stark abkühlt, da nur hier die Kälte entweichen kann. Die Kühlfläche 18 kann an eine zu behandelnde Stelle geführt und dort die Kälte, beispielsweise auf schmerzende Muskeln, Sehnen oder Bänder abgeben. Das Gas tritt nicht unmittelbar auf die zu behandelnde Stelle aus, sondern seitlich durch mehrere kleine Gasaustrittskanäle, die am Mantelumfang gleichabständig an der Unterseite des Aufnahmebehälters 14 angeordnet sind. Hierdurch wird vermieden, dass Gas unmittelbar auf die zu behandelnde Epidermis gelangt, so dass kein unangenehmer Kühleffekt auftritt und die Kühlung selektiv mittels Handführung dosiert werden kann. Durchstößt der Öffnungsstift 54 die Membran der Sahnekapsel 48, so tritt das Gas vollständig aus dem Gasdruckbehälter 16 aus, so dass eine einmalige Kühlwirkung erzielt werden kann. Nach einem Auswechseln des Gasdruckbehälters durch Herausschrauben des Aufnahmebehälters 14 aus dem Grundkörper 12 kann eine weitere Sahnekapsel aufgenommen und der Aufnahmebehälter 14 in den Grundkörper 12 eingesetzt werden, so dass durch ein Hineinschrauben der Deckkappe 56 in Richtung Handführungselement 78 eine weitere Kühlung bewirkt werden kann. Hierbei ist wesentlich, dass vor Auswechslung des Gasdruckbehälters 16 das Betätigungselement 38 wieder gegenüber dem Handführungselement 78 aufgeschraubt, bzw. zurückgesetzt wird, so dass nicht die Gefahr besteht, dass bei Einschrauben des Aufnahmebehälters 14 in den Grundkörper 12 ein sofortiger Gasaustritt erfolgt.

Des Weiteren ist denkbar, dass das Betätigungselement 38 ein Ventil umfasst, wobei beispielsweise beim Einsetzen des Aufnahmebehälters 14 in den Grundkörper 12 sofort die Verschlussmembran 44 des Gasdruckbehälter 16 durchstoßen wird und wobei dieser Durchstoßungsbereich gasdicht ausgebildet ist und erst bei Öffnen des im Betätigungselement angeordneten Verschlussventils ein Gasaustritt erfolgen kann. Alternativ kann der Gasdruckbehälter 16 ein kopfseitiges Ventil umfassen, wobei geeignete Betätigungsmittel innerhalb des Betätigungselements 38 angeordnet sind, die das Ventil zur Erzeugung eines Gasaustritts öffnend betätigen können. Die Verwendung eines Verschlussventils hat den Vorteil, dass insbesondere bei genügend großem Gasvolumen des Gasdruckbehälters 16 kein einmaliger Austritt des Gases erfolgt, sondern dosierbar eine mehrmalige Kühlungswirkung mit einstellbarer Kühltemperatur erreicht werden kann. Die Form, Anzahl und Durchmesser der Gasdurchtrittsblende 64 des Aufnahmeelements 60 bestimmen wesentlich die Geschwindigkeit mit der das Gas austreten kann und somit die zu erreichende Kühltemperatur. Dabei ist es denkbar, den Durchmesser der Gasdurchtrittsblende 64 oder die Anzahl der offenen Blenden 64 einstellbar zu machen, beispielsweise durch eine Rändelschraube und ein verschiebbares Blendenverdeckungselement, so dass Gasaustrittsvolumen und Geschwindigkeit und damit die zu erreichende Kühltemperatur von außen einstellbar sind. Zwischen den verschiedenen Verschraubungen und Verbindungselementen der Einzelteile der Kühlvorrichtung können gasdichte Dichtungen eingesetzt sein, die ein ungewolltes Ausströmen des Gases an den Verbindungs- bzw. Verschraubungsstellen verhindern können.

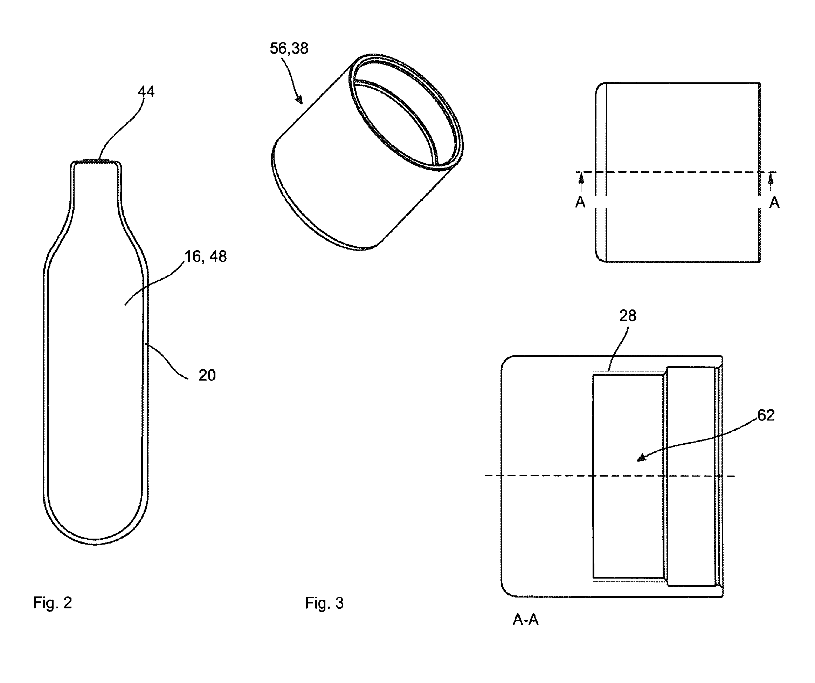

Fig. 2 stellt einen Schnitt durch einen Gasdruckbehälter 16, insbesondere durch eine Sahnekapsel 48 dar, die geeignet ist, in eine handgeführte Kühlvorrichtung 10 der Erfindung eingesetzt zu werden. Der Gasdruckbehälter 16 umfasst einen Gasdruckbehältermantel 20, der zumeist aus Metall insbesondere Aluminium besteht und eine hohe Wärmekapazität aufweist. Am Kopfende des Gasdruckbehälters 16 ist eine Verschlussmembran 44 aus dünnem Behältermantelmaterial angeordnet, die leicht mittels eines Öffnungsstifts 54 durchstoßen werden kann. Bei Austritt des unter Druck stehenden Gases aus dem Gasdruckbehälter, insbesondere $CO_2$ oder $N_2O$, entspannt sich das Gas, wobei die Temperatur abfällt. Somit erzeugt ausströmendes Gas eine Kältewirkung, wobei der Gasdruckbehältermantel stark abkühlt. Ist der Gasdruckbehälter 16 von isolierendem Material umgeben und nur an seinem bodenseitigen Ende bei eingesetztem Aufnahmebehälter von Aufnahmebehältermaterial umgeben, das gut wärmeleitfähig ist, so wird praktisch die gesamte erzeugte Kälte auf der Kühlfläche 18 der Bodenseite des Aufnahmebehälters 14 abgegeben. Hierdurch kann eine hohe Kühlwirkung und je nach Austrittsgeschwindigkeit und -Volumen des Gases angenehme 2 bis 5°C zur Behandlung von schmerzenden Körperstellen verwendet werden.

Fig. 3 zeigt in einer perspektivischen dreidimensionalen Darstellung sowie einer Seiten- und Schnittansicht A-A den Aufbau einer Deckkappe 56, die den Außenbereich des Betätigungselements 38 bildet. Die Deckkappe 56 weist ein einseitig geöffnetes axiales Ende auf, in dem ein Gewinde 28 in der Innenmantelfläche angebracht ist und das so ausgeformt ist, dass darin ein Verschlusselement 62 eingeschraubt werden kann.

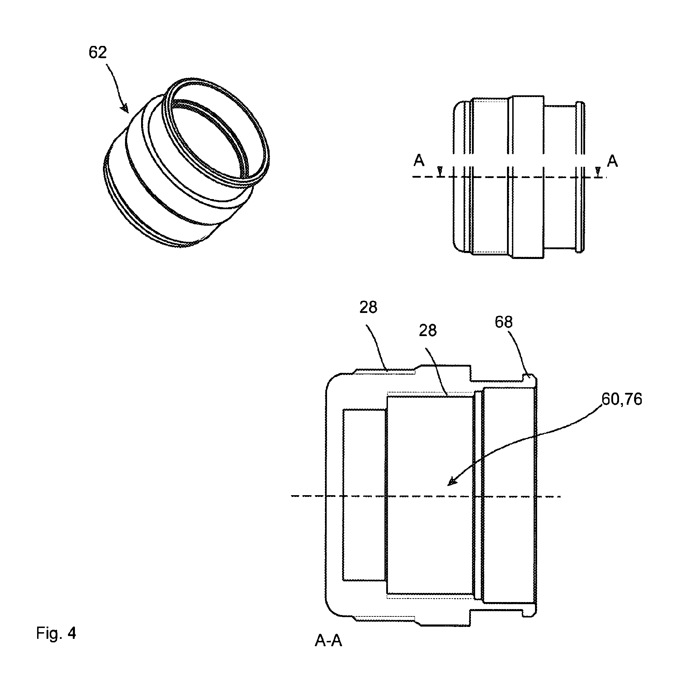

Fig. 4 zeigt in einer weiteren mehrperspektivischen Darstellung die Ausgestaltung eines Verschlusselements 62 des ersten Ausführungsbeispiels der Fig. 1. Das Verschlusselement 62 weist auf seiner Außenmantelfläche ein Feingewinde 28 auf, so dass es formkomplementär in die Deckkappe 56 eingeschraubt werden kann. Im Inneren weist das Verschlusselement 62 ein weiteres Feingewinde 28 auf, wobei ein Glockenelement 76 mit eingesetztem Öffnungsstiftelement 74 formkomplementär in eine Vertiefung eingesetzt bzw. eingepresst oder geklebt werden kann, sowie ein Aufnahmeelement 60 eingeschraubt werden kann. Des Weiteren weist es an seinem axial offenen Ende auf der Außenfläche einen Rastring 68 auf, der eine Rastnasenwirkung hervorruft und somit gegenüber dem Handführungselement 78 eine begrenzte Verfahrdistanz definiert.

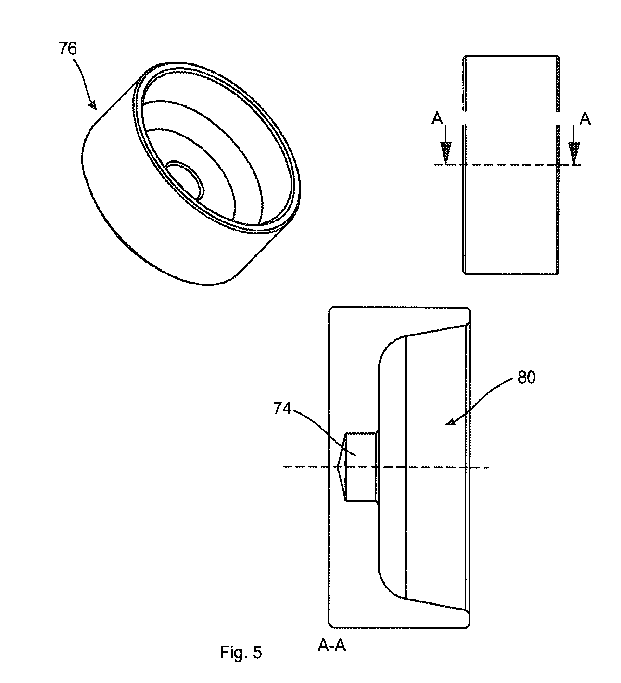

Fig. 5 zeigt sowohl perspektivisch als auch in einer Seiten- und Schnittansicht A-A eine Ausgestaltung eines Glockenelements 76. Das Glockenelement 76 ist wiederum axial halbseitig offen und umfasst einen Aufnahmebereich, in den ein Öffnungsstiftelement 74 eingesetzt werden kann und des Weiteren einen Glockenbereich, der zur Führung des austretenden Gases entlang dem Zwischenraum zwischen Gasdruckbehältermantel 20 und Innenoberfläche von Grundkörper 12 und Aufnahmebehälter 14 dient. Das Glockenelement 76 kann beispielsweise formschlüssig mittels Presspassung in das Verschlusselement 62 eingesetzt oder eingeklebt werden.

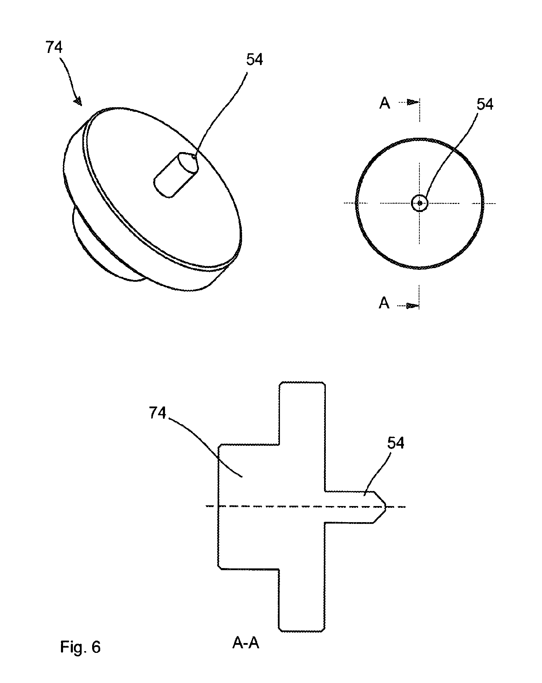

Schließlich zeigt **Fig. 6** perspektivisch sowie in einer Draufsicht und Seitenansicht ein Öffnungsstiftelement 74 des ersten Ausführungsbeispiels 10. Das Öffnungsstiftelement 74 weist auf seiner in Richtung Aufnahmebehälter 14 weisenden Seite einen Öffnungsstift 54 auf, der zum penetrierenden Durchstoßen einer Verschlussmembran 44 bzw. zum Betätigen eines Verschlussventils eines Gasdruckbehälters 16 ausgelegt ist. Dementsprechend ist das Material des Öffnungsstifts 74 aus einem robusten Werkstoff, beispielsweise Stahl ausgebildet, um den hohen austretenden Gasdruckkräfte sowie den großen Öffnungskräften standzuhalten und ein Abstumpfen des Öffnungsstifts 54 zu vermeiden. Öffnungsstiftelement 74, Glockenelement 76, Verschlusselement 72 sowie Deckkappe 56 bilden in ihrer zusammengesetzten Form das Betätigungselement 38 des Grundkörpers 12 des ersten Ausführungsbeispiels nach **Fig. 1.**

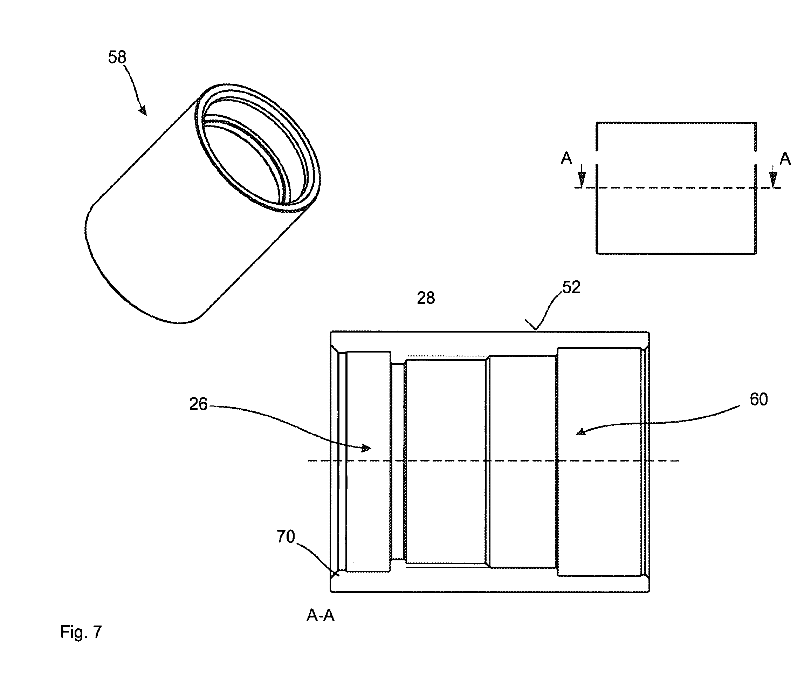

**Fig. 7** zeigt ein Griffstück 58, das den Handführungsbereich 52 des Handführungselements 78 umfasst. Das Griffstück 58 weist in seinem Inneren wiederum ein Feingewinde 28 zur Aufnahme des Aufnahmeelements 60 auf und weist entsprechende mehrstufige Radien zur Aufnahme von Aufnahmeelement 60 und zur Einsetzung des Betätigungselements 38 auf. Insbesondere weist es einen Rastring 70 auf, der als Rasthaken dient und eine Verfahrdistanz des Betätigungselements 38 gegenüber dem Handführungselement 78 begrenzt.

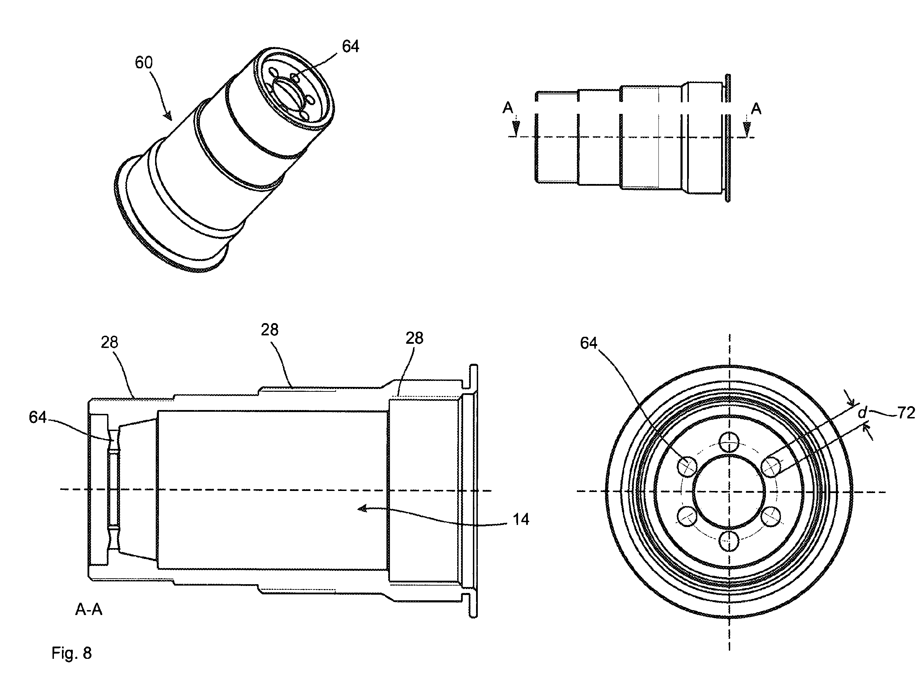

**Fig. 8** zeigt perspektivisch sowie in einer Drauf- und Seitenansicht und einer Schnittansicht eine Ausgestaltung eines Aufnahmeelements 60 des ersten Ausführungsbeispiels. Im Aufnahmeelement 60 kann ein Aufnahmebehälter 14 mit eingesetztem Gasdruckbehälter 16 eingeschraubt werden. Hierzu weist das Aufnahmeelement 60 ein Innengewinde 28 auf, sowie zwei weitere Außengewinde 28, die zum Einschrauben in das Griffstück 58 sowie zum aufgesetzten Einschrauben des Betätigungselements 38 dienen. Des Weiteren weist das Aufnahmeelement 60 an seiner axialen Oberseite mehrere Gasdurchtrittsblenden 64 auf, die einen Gasdurchtrittsblendendurchmesser $d$ 72 aufweisen. Die Anzahl sowie die Größe der Gasdurchtrittsblenden 64 bestimmen wesentlich die Ausströmgeschwindigkeit sowie Ausströmzeit des Gases aus dem eingesetzten Gasdruckbehälter 16 und bestimmen somit die erreichbare Kühltemperatur und Kühldauer. Beispielsweise kann eine variable Einstellbarkeit des Blendendurchmessers d 72 zur Einstellung der Kühltemperatur oder zur mehrmaligen Verwendung eines eingesetzten Gasdruckbehälters 16 variiert werden, wobei ein kleinerer Durchmesser d einer längeren Kühldauer d bei geringerer Temperatur entspricht.

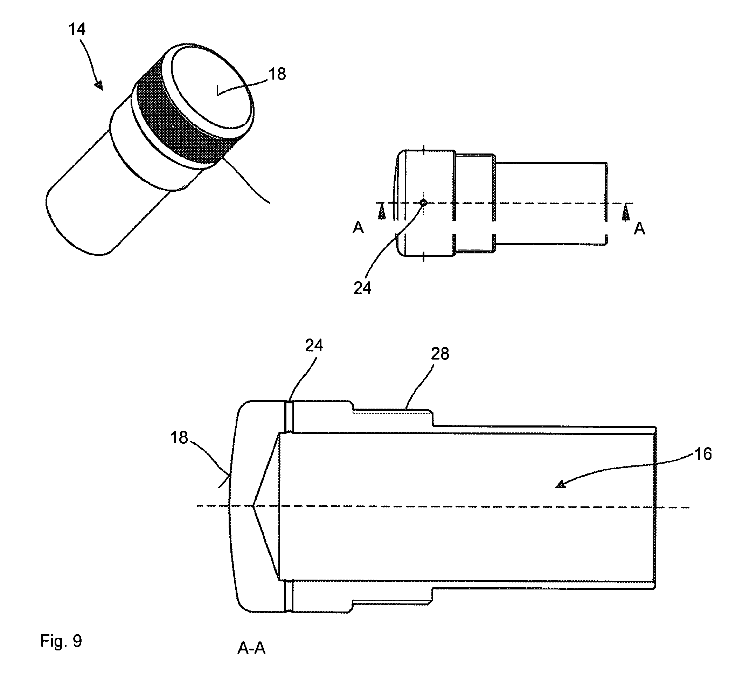

**Fig. 9 zeigt** sowohl in einer perspektivischen Darstellung als auch in einer Seiten- und Schnittdarstellung die Ausgestaltung eines Aufnahmebehälters 14, der in das erste Ausführungsbeispiel der **Fig. 1** mit aufgenommenem Gasdruckbehälter 16 eingesetzt werden kann. Im Gegensatz zu den vorher beschriebenen Teilen der Kühlvorrichtung, die aus gering wärmeleitfähigen bzw. isolierenden Materialien aufgebaut sind, ist der Aufnahmebehälter 14 aus gut leitendem, insbesondere metallischem Material aufgebaut, wobei insbesondere die Bodenseite mit außenseitig angeordneter Kühlfläche 18 aus besonders dickem, gut wärmeleitfähigem Material besteht, um eine hohe Wärmekapazität und Wärmeleitfähigkeit bereitzustellen. In das Innere des becherförmigen Aufnahmebehälters 14 kann ein Gasdruckbehälter 16 bodenseitig aufgenommen werden und mittels eines sich auf der Außenmantelfläche 28 des Aufnahmebehälters 14 befindlichen Feingewindes in den Grundkörper 12, insbesondere in das Innenfeingewinde des Aufnahmeelements 60, eingeschraubt werden. Bei Gasaustritt aus dem Gasdruckbehälter 16 wird das Gas entlang eines Spalts zwischen Gasdruckbehältermantel 20 und Innenfläche des Aufnahmebehälters 14 zu Gasaustrittskanälen 24 geführt, wo es in der Nähe der Kühlfläche 18 austreten kann, jedoch nicht die zu behandelnde Epidermis benetzt. Die Kühlfläche 18 kann insbesondere leicht gebogen konvex, aber auch flach oder konkav ausgeformt sein, je nach Eigenart der zu behandelnden Stelle, um eine möglichst hohe Kühlwirkung oder eine punktuelle Kühlung bewirken zu können. Statt einer Ableitung des Gases entlang eines Spalts zwischen Mantelaußenfläche 20 des Gasdruckbehälters 16 und Innenwandung des Aufnahmebehälters 14 können zusätzliche Gasführungskanäle im Inneren von Grundkörper 12 und/oder Aufnahmebehälter 14 angeordnet sein, die eine Ableitung des Gases sowie einen Gasaustritt ermöglichen.

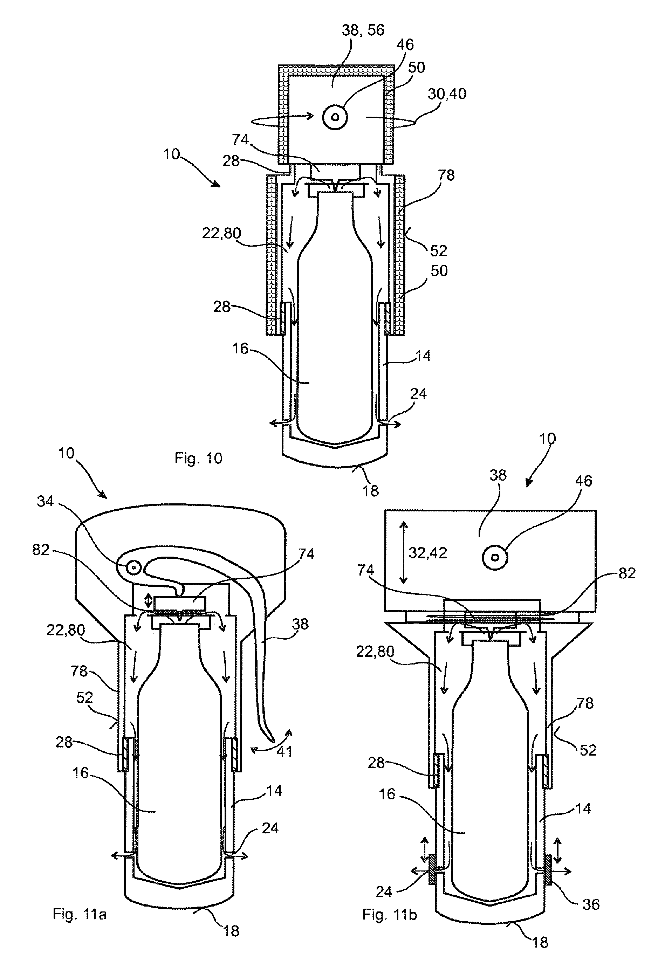

**Fig. 10** zeigt schematisch ein weiteres Ausführungsbeispiel einer handgeführten Kühlvorrichtung 10. Grundsätzlich entspricht der Aufbau dem des in **Fig. 1** dargestellten ersten Ausführungsbeispiels 10, jedoch sind Handführungsbereich 52 und Deckkappe 56 des Betätigungselements 38 zusätzlich mit einem wärmeisolierenden Material 50 überzogen, um einen ungewollten Kälteaustritt am Grundkörper 12 zu verhindern. Das Betätigungselement 38 wird mittels einer Drehmechanik 30, die infolge einer Drehbewegung durch einen Öffnungsstift 54 eines Öffnungsstiftelements 74 eine Verschlussmembran 44 eines Gasdruckbehälters 16 durchstößt, aktiviert, wobei Gas austritt, entlang des Außenmantels 20 des Gasdruckbehälters 16 strömt und an Gasaustrittskanälen 24 am bodenseitigen Ende des Aufnahmebehälters 14 austritt. Durch die Wärmeisolation 50 wird die gesamte abgegebene Kälte im unisolierten, gut wärmeleitfähigen Bereich der Kühlfläche 18 abgegeben und kann zur Kühlung einer schmerzenden Stelle verwendet werden. Zur Sicherung einer ungewollten Aktivierung der Kühlvorrichtung 10 ist ein Sicherungselement 46, beispielsweise ein Sicherungsstift der herausgezogen werden muss, oder ein Sicherungsdruckknopf, der hineingedrückt werden muss, bevor eine Drehbewegung 40 der Drehmechanik 30 erfolgen kann, am Betätigungselement 38 angeordnet.

**Fig. 11** zeigt zwei weitere Ausführungsbeispiele einer handgeführten Kühlvorrichtung 10, die in ihrem grundsätzlichen Aufbau und Wirkungsweise den in **Fig. 1** bzw. **Fig. 10** dargestellten Ausführungsbeispielen gleichen. Somit wird auf die Beschreibung der gleichartigen Komponenten verzichtet. In **Fig. 11a** ist als Betätigungselement 28 eine Wippmechanik 34 vorgesehen, die mittels einer Wippdruckbewegung 41 eines Wipphebels ein penetrierendes Durchstoßen einer Verschlussmembran 44 eines Gasdruckbehälters 16 mittels des Öffnungsstiftelements 74 bewirkt. Bei Entlastung des Wipphebels des Betätigungselements 38 wird mittels eines Federmittels 82 eine Rückstellung bewirkt, so dass das Gas ungehindert ausströmen kann. Das Ausströmen des Gases sowie der Aufbau des Grundkörpers 12 und des Aufnahmebehälters 14 entspricht dabei den in den vorangegangenen Ausführungsbeispielen beschriebenen Ausführungen.

Demgegenüber ist in **Fig. 11b** ein weiteres Ausführungsbeispiel dargestellt, in dem das Betätigungselement 38 mittels einer axialen Druckbewegung 42 ein Durchstoßen der Verschlussmembran 44 des Gasdruckbehälters 16 bewirkt. Auch in diesem Fall stellt ein Federmittel 82 eine Rückstellung des Betätigungsmittels 38 sicher, so dass das Gas ungehindert aus der penetrierten Verschlussmembran austreten kann. In diesem Fall ist ein Verschlussventil 36 an den Gasaustrittskanälen 24 angeordnet, durch deren Öffnen oder Verschließen ein Gasaustritt ermöglicht oder verhindert werden kann. Hierzu ist es notwendig, das alle Verschraubungselemente und sonstigen Fügeelemente der Kühlvorrichtung 10 gasdicht ausgelegt, beispielsweise mittels Dichtungen gasdicht ausgelegt sind, um den im Inneren herrschenden Druck nicht ungewollt ausweichen zu lassen. Durch Öffnen oder Verschließen des Verschlussventils 36 kann dosierend oder mehrmalig eine Kühlwirkung an der Kühlfläche 18 erzeugt werden. Zum Sichern einer ungewollten Aktivierung des Betätigungselements 38 ist ein Sicherungselement 46 an der Kappe 56 angeordnet, wobei erst bei Betätigung des Sicherungselements 46, beispielsweise beim Herausziehen eines Sicherungsstifts oder beim Hineindrücken eines Sicherungsbolzens eine Druckbewegung auf das Betätigungselement 38 ausgeübt werden kann, um die Membran 44 des Gasdruckbehälters 16 zu öffnen.

Die Erfindung schlägt eine handgeführte Kühlvorrichtung vor, die mobil einsetzbar ist, eine falsche Handhabung verhindert, eine konstante Kühlwirkung erzeugt und dabei platzsparend und kompakt für eine einmalige Anwendung ausgelegt werden kann. Selbst Ungeübte können eine therapeutische Kühlwirkung erzeugen, ohne eine Kälteverbrennung der zu behandelnden Bereiche hervorzurufen. Die Erfindung verwendet eine Standardgasdruckpatrone, beispielsweise eine in großen Mengen und zu geringen Kosten verfügbare Sahnekapsel, bei der durch Abschrauben des Aufnahmebehälters, dem Einsetzen der Gaspatrone in den Aufnahmebehälter, Einschraubens des Aufnahmebehälters mit eingelegter Patrone in den Grundkörper, Verdrehen des Grundkörpers über eine Gewindekappe und damit Eindrücken eines Stahlstiftes in die Gasdruckpatrone ein Gas ausströmt, wobei das entweichende und expandierende Gas im Endbereich des Aufnahmebehälters eine Kühlwirkung erzeugt, so dass die Kühlfläche auf die zu behandelnde Stelle eine Kühlwirkung ausüben kann. Die erfindungsgemäße Kühlvorrichtung eignet sich insbesondere zum Mitführen und zur mobilen ambulanten Behandlung bei Sportverletzungen oder in anderen vergleichbaren Fällen, in denen eine rasche Kühlwirkung ohne geschultes Fachpersonal erzielt werden soll. Insbesondere kann die Kühlvorrichtung so ausgelegt sein, das nach Öffnen des Gasdruckbehälters in ca. 5 bis 10 Sekunden eine Kühlwirkung bei etwa 5 bis 7°C erreicht werden kann, wobei diese Temperatur über einen Zeitraum von 3 bis 5 Minuten aufrecht erhalten werden kann. Die Kühlvorrichtung ist dabei sehr klein ausgelegt, kaum größer als die Abmessungen des Gasdruckbehälters selbst und vermeidet jede Gefahr einer Fehlbedienung oder von Kälteverbrennungen. Es können jegliche Arten von Druckluft-, $N_2O$-, $CO_2$- oder sonstige gasbeinhaltende Gasdruckbehälter verwendet werden.

**Patentansprüche**

1. Handgeführte Kühlvorrichtung (10) zur Kryotheraphie für die Behandlung von Schmerzen, Entzündungen, Muskelverspannungen oder dergleichen, umfassend einen Grundkörper (12) und einen in den Grundkörper (12) zumindest teilweise einsetzbaren Aufnahmebehälter (14) zur auswechselbaren Aufnahme und Einsetzung eines Gesdruckbehälters (16), wobei der Grundkörper (12) aus einem Material niedriger spezifischer Wärmeleitfähigkeit besteht und ein Gasaustrittsbetätigungsmittel (26) umfasst, dass manuell betätigbar ein Ausströmen eines Gases aus dem Gasdruckbehälter (16) bei eingesetztem Aufnahmebehälter (14) bewirken kann, **gekennzeichnet dadurch, dass** der Aufnahmebehälter (14) aus einem Material hoher spezifischer Wärmeleitfähigkeit besteht und eine Kühlfläche (18) umfasst, die durch die Expansionswirkung des ausströmenden Gases im Inneren der Kühlvorrichtung (10) sowie durch die Abkühlung des Gasdruckbehältermantels (20) kühlbar ist, wobei das Gas innerhalb des Grundkörpers (12) und des Aufnahmebehälters (14) mittels Gasführungsmittel geführt und am Aufnahmebehälter (14) seitlich benachbart zur Kühlfläche (18) und derart von der Kühlfläche weggeleitet austreten kann, dass die zu kühlende Fläche nicht unmittelbar begast bzw. besprüht wird.

2. Kühlvorrichtung (10) nach Anspruch 1, **dadurch gekennzeichnet, dass** der Aufnahmebehälter (14) im Wesentlichen becherförmig und formkomplementär zu dem Bodenbereich des Gasdruckbehälter (16) ausgestaltet ist und aus einem Material hoher Wärmeleitfähigkeit, bevorzugt Metall, besteht, wobei die Bodenaußenfläche des Aufnahmebehälters (14) die Kühlfläche (18) ausbildet und der Aufnahmebehälter (14) zumindest einen, insbesondere mehrere Gasaustrittskanäle (24) entlang eines Umfangbereichs am bodenseitigen Ende umfasst, und der Aufnahmebehälter (14) den Gasdruckbehälter (16) zumindest Im Bodenbereich im Wesentlichen formschlüssig zur Erzielung einer Wärmekopplung aufnehmen kann, wobei zwischen Innenwand des Aufnahmebehälters (14) und äußerem Gasdruckbehältermantel (20) ausströmendes Gas vom offenen, im Grundkörper eingesetzten Ende des Aufnahmebehälters (14) bis zu dem Gasaustrittskanal (24), bevorzugt entlang von einem oder mehreren Gasführungsmitteln (22), insbesondere Gasführungskanälen, strömen kann.

3. Vorrichtung nach Anspruch 1 oder 2,
**dadurch gekennzeichnet,**
**dass** der Aufnahmebehälter (14) mit aufgenommenen Gasdruckbehälter (16) in den Grundkörper (12) einschraubbar, einrastbar oder bajonettverschlussartig einsetzbar ist.

4. Vorrichtung nach einem der vorangegangenen Ansprüche,
**dadurch gekennzeichnet,**
**dass** das Gasaustrittsbetätigungsmittel (26) eine Dreh- (30), Wipp- (34), Druck-(32) oder Zug-Austrittsmechanik zum Bewirken eines Gasaustritts aus dem Gasdruckbehälter (16) umfasst, wobei nach eingesetztem Gasdruckbehälter (16) durch eine Dreh-, Wipp-, Druck- oder Zugbewegung eines Betätigungselements (38) die Austrittsmechanik ein Verschlussventil (36) des Gasdruckbehälters (16) oder des Grundkörpers (12) betätigen kann, oder eine Verschlussmembran (44) des Gasdruckbehälters (16) durchstoßen kann.

5. Vorrichtung nach Anspruch 4,
**dadurch gekennzeichnet,**
**dass** das Gasaustrittsbetätigungsmittel (26) einen Öffnungsstift (54) zum penetrierenden Durchstoßen der Verschlussmembran (44) des Gasdruckbehälters (16) umfasst.

6. Vorrichtung nach Anspruch 3,
**dadurch gekennzeichnet,**
**dass** der Grundkörper (12) zumindest eine Gasdurchtrittsblende (64) umfasst, die ausgelegt ist, eine Gasaustrittsgeschwindigkeit zu drosseln, wobei bevorzugt die Gasdurchtrittsblende (64) einen einstellbaren Blendendurchmesser d (72) aufweist, so dass die Gasaustrittsgeschwindigkeit und somit die erwünschte Kühltemperatur bevorzugt im Bereich zwischen 2 bis 12°C, insbesondere 5 bis 7°C, einstellbar ist.

7. Vorrichtung nach einem der vorangegangenen Ansprüche,
**dadurch gekennzeichnet,**
**dass** die Austrittsmechanik (30, 32, 34) ein Sicherungselement (46) umfasst, das ein ungewolltes Betätigen des Betätigungselements (38) verhindern kann.

8. Vorrichtung nach einem der vorangegangenen Ansprüche,
**dadurch gekennzeichnet,**
**dass** der Aufnahmebehälter (14) geeignet ist, einen $N_2O$- oder $CO_2$-Gasdruckbehälter mit einen Füllgewicht von 2g bis 350g, bevorzugt von 6g bis 20g, insbesondere von 8g, aufzunehmen, der eine Längenabmessung von 4 bis 20cm, insbesondere 6 bis 7cm, und einen Durchmesser von 1 bis 7cm, insbesondere 1 bis 2,5cm, aufweist.

9. Vorrichtung nach Anspruch 8,
**dadurch gekennzeichnet,**
**dass** der Gasdruckbehälter (16) eine handelsübliche $CO_2$-8g-Sahnespenderpatrone (48), insbesondere eine ISI-Sahnekapsel ist.

10. Vorrichtung nach einem der vorangegangenen Ansprüche,
**dadurch gekennzeichnet,**
**dass** zumindest die Kühlfläche (18) des Aufnahmebehälters (14) aus einem Werkstoff hoher spezifischer Wärmeleitfähigkeit, bevorzugt Metall, insbesondere einer Aluminium-Legierung, wie AlMgSi, besteht, und der Grundkörper (12) sowie die übrigen Teile aus einem Material niedriger spezifischer Wärmeleitfähigkeit, insbesondere wärmeisolierenden Kunststoff, bevorzugt POM (Polyoxymethylen), bestehen, wobei bevorzugt ein Handführungsbereich (52) des Grundkörpers (12) zusätzlich durch eine Isolationsschicht (50) wärmeisolierend ausgestaltet ist.

11. Vorrichtung nach Anspruch 5,
**dadurch gekennzeichnet,**
**dass** der Öffnungsstift (54) von einem Öffnungsstiftelement (74) umfasst ist, das aus Stahl, insbesondere X20Cr13, besteht.

12. Vorrichtung nach einem der vorangegangenen Ansprüche,
**dadurch gekennzeichnet,**
**dass** der Grundkörper (12) mehrteilig ausgeführt ist und insbesondere eine Deckkappe (56), ein Griffstück (58), ein Aufnahmeelement (60), ein Verschlusselement (62), ein Glockenelement (76) und ein Öffnungsstiftelement (74) umfasst, wobei zumindest die Deckkappe (56), das Verschlusselement (62), das Glockenelement (76) und das Offnungsstiftelement (74) formschlüssig zur Ausbildung eines Gasaustrittsbetätigungsmittels (26) miteinander verbunden sind, sowie zumindest Griffstück (58) und Aufnahmeelement (60) formschlüssig miteinander zur Ausbildung eines Handführungselements (78) verbunden sind, das zur Aufnahme des Aufnahmebehälters (14) und zur Handführung der Kühlvorrichtung (10) durch einen Benutzer dient, und das Gasaustrittsbetätigungsmittel (26) gegenüber dem Handführungselement (78) verschieblich, insbesondere verdrehbar, zur Ausübung einer Betätigungsbewegung (40, 41, 42) für eine penetrierende Durchstoßung einer Verschlussmembran (44) eines aufgenommenen Gasdruckbehälters (16) gelagert ist.

## Claims

1. Manually guided cooling device (10) for cryotherapy to treat pain, inflammation, muscle tension or the like, comprising a basic element (12) and a receiving container (14) at least partially insertable into said basic element (12) for replaceably holding and inserting a pressurized gas container (16), where said basic element (12) consists of a material of low specific thermal conductivity and comprises a gas outlet actuating means (26), that with manual actuation can effect an outflow of a gas from the pressurized gas container (16) when the receiving container (14) is inserted,
   **characterized in that**
   the receiving container (14) consists of a material of high specific thermal conductivity and comprises a cooling surface (18) which can be cooled by the expansion effect of the outflowing gas in the interior of the cooling device (10) and by the cooling down of the pressurized gas container jacket (20), where the gas inside the basic element (12) and the receiving container (14) is guided by means of gas guidance means and can exit the receiving container (14) laterally adjacent to the cooling surface (18) and directed away from the cooling surface such that the area to be cooled is not directly exposed to gas or spray.

2. Cooling device (10) according to claim 1,
   **characterized in**
   **that** the receiving container (14) is designed substantially cup-like and complementary in shape to the bottom area of the pressurized gas container (16) and consists of a material of high thermal conductivity, preferably metal, where the outer bottom surface of the receiving container (14) forms the cooling surface (18) and the receiving container (14) comprises at least one and in particular several gas outlet ducts (24) along a circumferential area on the bottom end, and in that the receiving container (14) can receive the pressurized gas container (16) at least in the bottom area in a substantially positive connection in order to obtain a thermal coupling, where gas flowing out between the inner wall of the receiving container (14) and the outer pressurized gas container jacket (20) can flow from the open end, placed inside the basic element, of the receiving container (14) to the gas outlet duct (24), preferably along one or more gas guidance means (22), in particular gas guidance ducts.

3. Device according to claim 1 or 2,
   **characterized in**
   **that** the receiving container (14) with the received pressurized gas container (16) can be inserted into the basic element (12) by a screw-in, snap-in or bayonet-type connection.

4. Device according to one of the preceding claims,
   **characterized in**
   **that** the gas outlet actuating means (26) comprises a rotary (30), lever (34), press (32) or pull-type outlet mechanism to effect a gas outlet from the pressurized gas container (16), where after insertion of the pressurized gas container (16) the outlet mechanism can, by a rotary, lever, press or pull movement of an actuating element (38), operate a shut-off valve (36) of the pressurized gas container (16) or of the basic element (12), or can puncture a sealing membrane (44) of the pressurized gas container (16).

5. Device according to claim 4,
   **characterized in**
   **that** the gas outlet actuating means (26) comprises an opening pin (54) for penetrative puncturing of the sealing membrane (44) of the pressurized gas container (16).

6. Device according to claim 3,
   **characterized in**
   **that** the basic element (12) comprises at least one gas outlet aperture (64) which is designed to restrict a gas outlet speed, where the gas outlet aperture (64) preferably has a settable aperture diameter d (72) such that the gas outlet speed and hence the required cooling temperature is settable preferably in the range between 2 and 12°C, in particular 5 and 7°C.

7. Device according to one of the preceding claims,
   **characterized in**
   **that** the outlet mechanism (30, 32, 34) comprises a safety element (46) that can prevent an inadvertent operation of the actuating element (38).

8. Device according to one of the preceding claims,
   **characterized in**
   **that** the receiving container (14) is suitable for re-

ceiving an $N_2O$ or $CO_2$ pressurized gas container with a filled weight from 2 g to 350 g, preferably from 6 g to 20 g, in particular from 8 g, which has a length dimension from 4 to 20 cm, in particular 6 to 7 cm, and a diameter from 1 to 7 cm, in particular 1 to 2.5 cm.

9. Device according to claim 8,
**characterized in**
**that** the pressurized gas container (16) is a commercially available $CO_2$ 8 g whipped-cream dispenser cartridge (48), in particular an ISI cream capsule.

10. Device according to one of the preceding claims,
**characterized in**
**that** at least the cooling surface (18) of the receiving container (14) consists of a material of high specific thermal conductivity, preferably metal, in particular an aluminium alloy such as AlMgSi, and the basic element (12) and the other parts consist of a material of low specific thermal conductivity, in particular thermally insulating plastic, preferably POM (polyoxymethylene), where a manual guidance area (52) of the basic element (12) is preferably additionally designed with thermal insulation provided by an insulating layer (50).

11. Device according to claim 5,
**characterized in**
**that** the opening pin (54) is enclosed by an opening pin element (74) that consists of steel, in particular X20Cr13.

12. Device according to one of the preceding claims,
**characterized in**
**that** the basic element (12) is designed in several parts and in particular comprises a cover cap (56), a grip section (58), a receiving element (60), a shut-off element (62), a bell-shaped element (76) and an opening pin element (74), where at least the cover cap (56), the shut-off element (62), the bell-shaped element (76) and the opening pin element (74) are positively connected to one another to form a gas outlet actuating means (26), and at least the grip section (58) and the receiving element (60) are positively connected to one another to form a manual guide element (78) which serves to receive the receiving container (14) and to allow manual guidance of the cooling device (10) by a user, and in that the gas outlet actuating means (26) is mounted movably, in particular rotatably, relative to the manual guide element (78) in order to perform an actuating movement (40, 41, 42) for penetrative puncturing of a sealing membrane (44) of a received pressurized gas container (16).

**Revendications**

1. Dispositif refroidissant (10) à commande manuelle, destiné à la cryothérapie pour le traitement de douleurs, d'inflammations, de tensions musculaires ou similaires, comprenant un corps de base (12) et un récipient récepteur (14) insérable au moins partiellement dans le corps de base (12) pour la réception et la mise en place interchangeables d'un réservoir de gaz sous pression (16), sachant que le corps de base (12) est constitué d'un matériau à faible conductivité thermique et comprend un moyen d'actionnement de sortie de gaz (26), qui peut provoquer par actionnement manuel une émission d'un gaz hors du réservoir de gaz sous pression (16) lorsque le récipient récepteur (14) est inséré,
**caractérisé en ce**
**que** le récipient récepteur (14) est constitué d'un matériau à haute conductivité thermique et présente une surface refroidissante (18) qui peut être refroidie par la détente du gaz émis à l'intérieur du dispositif refroidissant (10) ainsi que par le refroidissement de la paroi du réservoir de gaz sous pression (20), sachant qu'à l'intérieur du corps de base (12) et du récipient récepteur (14), le gaz est guidé par des moyens de guidage et peut s'échapper dans le récipient récepteur (14) latéralement au voisinage de la surface refroidissante (18) en s'éloignant de la surface refroidissante de manière telle que la surface à refroidir n'est pas directement gazée ou aspergée.

2. Dispositif refroidissant (10) selon la revendication 1,
**caractérisé en ce**
**que** le récipient récepteur (14) est pour l'essentiel conçu en forme de godet et en complémentarité de forme avec la zone du fond du réservoir de gaz sous pression (16), et est constitué d'un matériau à haute conductibilité thermique, d'un métal de préférence, sachant que la face extérieure du fond du récipient récepteur (14) forme la surface refroidissante (18) et que le récipient récepteur (14) comprend au moins un, en particulier plusieurs conduits de sortie de gaz (24) le long d'une zone circonférentielle sur l'extrémité côté fond, que le récipient récepteur (14) peut, au moins dans la zone du fond, loger le réservoir de gaz sous pression (16) par complémentarité de forme pour l'essentiel afin d'obtenir un couplage thermique, et que le gaz émis entre la paroi intérieure du récipient récepteur (14) et la paroi extérieure du réservoir de gaz sous pression (20) peut s'écouler de l'extrémité ouverte du récipient récepteur (14) insérée dans le corps de base jusqu'au conduit de sortie de gaz (24), de préférence le long d'un ou plusieurs moyens de guidage (22), en particulier de conduits de guidage de gaz.

3. Dispositif selon la revendication 1 ou 2,
**caractérisé en ce**

**que** le récipient récepteur (14) muni du réservoir de gaz sous pression (16) peut être fixé dans le corps de base (12) par vissage, encliquetage ou emboîtement de type à baïonnette.

4. Dispositif selon l'une des revendications précédentes,
   **caractérisé en ce**
   **que** le moyen d'actionnement de sortie de gaz (26) comprend un mécanisme d'échappement par rotation (30), basculement (34), pression (32) ou traction afin de provoquer une sortie de gaz du réservoir de gaz sous pression (16), sachant qu'après insertion du réservoir de gaz sous pression (16) et par un mouvement de rotation, de basculement, de pression ou de traction d'un élément d'actionnement (38), le mécanisme d'échappement peut actionner un clapet d'obturation (36) du réservoir de gaz sous pression (16) ou du corps de base (12), ou transpercer une membrane d'obturation (44) du réservoir de gaz sous pression (16).

5. Dispositif selon la revendication 4,
   **caractérisé en ce**
   **que** le moyen d'actionnement de sortie de gaz (26) comprend une broche d'ouverture (54) destinée au perçage par pénétration de la membrane d'obturation (44) du réservoir de gaz sous pression (16).

6. Dispositif selon la revendication 3,
   **caractérisé en ce**
   **que** le corps de base (12) comprend au moins un orifice de passage de gaz (64) dimensionné pour réduire une vitesse de sortie du gaz, sachant que de préférence l'orifice de passage de gaz (64) présente un diamètre d'ouverture d (72) réglable, de sorte que la vitesse de sortie du gaz est réglable et qu'ainsi la température de refroidissement souhaitée peut être réglée de préférence dans la plage de 2 à 12 °C, en particulier entre 5 et 7 °C.

7. Dispositif selon l'une des revendications précédentes,
   **caractérisé en ce**
   **que** le mécanisme d'échappement (30, 32, 34) comprend un élément de sûreté (46) qui peut empêcher un actionnement involontaire de l'élément d'actionnement (38).

8. Dispositif selon l'une des revendications précédentes,
   **caractérisé en ce**
   **que** le récipient récepteur (14) convient pour loger un réservoir de gaz $N_2O$ ou $CO_2$ sous pression avec un poids de remplissage de 2 g à 350 g, de préférence de 6 g à 20 g, en particulier de 8 g, et qui présente une étendue en longueur de 4 à 20 cm, en particulier de 6 à 7 cm, et un diamètre de 1 à 7 cm, en particulier de 1 à 2,5 cm.

9. Dispositif selon la revendication 8,
   **caractérisé en ce**
   **que** le réservoir de gaz sous pression (16) est une cartouche distributrice de crème chantilly à 8 g de $CO_2$ (48) en vente dans le commerce, en particulier une capsule de crème chantilly ISI.

10. Dispositif selon l'une des revendications précédentes,
    **caractérisé en ce**
    **qu'**au moins la surface refroidissante (18) du récipient récepteur (14) est constituée d'un matériau à haute conductivité thermique, d'un métal de préférence, en particulier d'un alliage d'aluminium, par exemple AlMgSi, et que le corps de base (12) ainsi que les autres pièces sont constitués d'un matériau à faible conductivité thermique, en particulier d'une matière synthétique thermo-isolante, de préférence de POM (polyoxyméthylène), sachant que de préférence une zone de guidage manuel (52) du corps de base (12) est en outre rendue thermo-isolante par une couche isolante (50).

11. Dispositif selon la revendication 5,
    **caractérisé en ce**
    **que** la broche d'ouverture (54) est entourée d'un élément à broche d'ouverture (74) qui est constitué d'acier, en particulier de X20Cr13.

12. Dispositif selon l'une des revendications précédentes,
    **caractérisé en ce**
    **que** le corps de base (12) est réalisé en plusieurs parties et comprend en particulier un capuchon (56), une poignée (58), un élément récepteur (60), un élément obturateur (62), un élément en cloche (76) et un élément à broche d'ouverture (74), sachant qu'au moins le capuchon (56), l'élément obturateur (62), l'élément en cloche (76) et l'élément à broche d'ouverture (74) sont reliés entre eux par complémentarité de forme pour former un moyen d'actionnement de sortie de gaz (26), et qu'au moins la poignée (58) et l'élément récepteur (60) sont reliés entre eux par complémentarité de forme pour former un élément de guidage manuel (78) qui sert à loger le récipient récepteur (14) et au guidage manuel du dispositif refroidissant (10) par un utilisateur, et que le moyen d'actionnement de sortie de gaz (26) est logé de manière déplaçable par rapport à l'élément de guidage manuel (78), en particulier par rotation, afin d'exercer un mouvement d'actionnement (40, 41, 42) pour un perçage par pénétration d'une membrane d'obturation (44) d'un réservoir de gaz sous pression (16) inséré.

Fig. 1

44

16, 48

20

56,38

28

62

A

A

A-A

Fig. 2

Fig. 3

EP 2 558 013 B1

62

A A

28

28

68

60,76

A-A

Fig. 4

17

Fig. 5

A-A

EP 2 558 013 B1

54

54

74

54

74

A-A

Fig. 6

Fig. 7

72

d

64

14

28

28

28

64

60

64

A-A

Fig. 8

Fig. 9

Fig. 10

Fig. 11a

Fig. 11b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 60109801 T2
- US 20090248001 A1
- DE 69918810 T2
- DE 19548652 A1
- DE 20305694 U1
- US 2536001 A
- DE 19548652 C
- DE 20305694 U
- US 2008142036 A